# EUROPÄISCHE PATENTSCHRIFT

(11) **EP 2 027 464 B1**
(45) Veröffentlichungstag und Bekanntmachung des Hinweises auf die Patenterteilung: **28.07.2010**
(21) Anmeldenummer: 07725256.7
(22) Anmeldetag: 16.05.2007
(51) Int. Cl.: G01N 33/50

(54) **VERFAHREN ZUM NACHWEIS DER VIABILITÄT**
METHOD FOR DETECTING VIABILITY
PROCÉDÉ DE DÉTERMINATION DE LA VIABILITÉ

(30) Priorität: 19.05.2006 DE 102006023906
(43) Veröffentlichungstag der Anmeldung: 25.02.2009
(73) Patentinhaber: Ovamed GmbH, 22885 Barsbüttel (DE)
(72) Erfinder: ABROMEIT, Norbert, 22926 Ahrensburg (DE)
(74) Vertreter: Dinné, Erlend
(86) Internationale Anmeldenummer: PCT/EP2007/004340
(87) Internationale Veröffentlichungsnummer: WO 2007/134761

(56) Entgegenhaltungen:
- WO-A-99/33479
- BLACK M I ET AL: "SURVIVAL RATES OF PARASITE EGGS IN SLUDGE DURING AEROBIC AND ANAEROBIC DIGESTION" APPLIED AND ENVIRONMENTAL MICROBIOLOGY, Bd. 44, Nr. 5, 1982, Seiten 1138-1143, XP002446722 ISSN: 0099-2240

## Beschreibung

Die Erfindung betrifft ein Verfahren zum Nachweis der Viabilität von *Trichuris suis -* Eiern.

*Trichuris suis* (Peitschenwurm des Schweins) gehört zur Gruppe der Rundwürmer (Nematoden), die als Parasit im Schweinedarm leben, und ist pharmakologisch insbesondere von Interesse für die Behandlung exzessiver Immunreaktionen, wie dies in WO 99/33479 beschrieben ist: die Gewinnung von *Trichuris suis-*Eiern wird beschrieben, ebenso die Besiedlung und Behandlung des Darms von Human-Patienten mit fortgeschrittenem, medikations-resistentem *Morbus Crohn* mit *Trichuris suis.*

Für solchen und anderen Einsatz ist es von Interesse, die Viabilität der eingesetzten *Trichuris suis -* Eier zu kennen. Dafür werden bislang von *Trichuris suis* freie Schweine mit *Trichuris suis-Eiern,* dem pharmazeutisch wirksamen Bestandteil, besiedelt, die so behandelten Schweine werden dann geschlachtet und der Darm wird auf *Trichuris suis* untersucht.

Nachteilig an diesem Vorgehen ist der große Aufwand hinsichtlich *Trichuris suis* freier Schweine, deren Zucht und Unterbringung sowie deren Schlachten. Nachteilig ist auch die lange Dauer einer solchen Untersuchung von *Trichuris suis-* Eiern, die so auf Viabilität überprüft werden können.

Aufgabe der Erfindung war es, hier Abhilfe zu schaffen, insbesondere ein Verfahren zum Nachweis der Viabilität von *Trichuris suis -* Eiern zu schaffen, das die Nachteile des Standes der Technik nicht oder zumindest nicht in dem Maße aufweist, das insbesondere *in vitro* durchgeführt werden kann, wirtschaftlich, standardisiert und von angelerntem Personal.

Gelöst wird diese Aufgabe durch ein Verfahren zum Nachweis der Viabilität von *Trichuris suis -* Eiern, wie dies in den Patentansprüchen näher gekennzeichnet ist, und das dadurch gekennzeichnet ist, dass man den Durchgang der Eier durch die Magen-Darm-Passage eines Schweins *in vitro* simuliert hinsichtlich zeitlicher Abfolge und Milieu, nämlich zunächst das Magen-Milieu und anschließend in absteigender Enzym- und Salz-Konzentration das Darm-Milieu, und damit die Larven aus den Eiern zum Schlüpfen bringt.

Die Simulation der Magen-Darm-Passage eines Schweins erfolgt durch Zugabe von entsprechenden Enzymen und Salzen und Inkubation bei erhöhter Temperatur, wobei im einzelnen vorteilhaft folgende Parameter eingestellt werden:
- Enzyme, in der Darmsimulation mit abnehmender Konzentration,
- pH-Wert,
- Wärme,
- evtl. unterschiedliche Gaskonzentration im Klimaschrank,
- Rüttelbewegungen,
- Salzlösungen, in der Darmsimulation mit abnehmender Konzentration.

Damit kann das erste Larvalstadium in einem flüssigen oder halbfesten Milieu, insbesondere in einer Lösung, zum Schlupf gebracht werden.

Grundsätzlich sind mit Enzyme alle Verdauungsstoffe, Enzyme und Enzymmischungen gemeint, die wie Darmenzyme wirken und allgemein abbauende oder verändernde Eigenschaften des Magen-Darminhalts (Magen-Darmreaktionen) hervorrufen. Eine über die Zeit abnehmende Enzymkonzentration ist beim Schlupf ein wichtiger auslösender Faktor.

Bei der Simulation des Magens wird bevorzugt 1-5% (alle % Angaben sind Gewichts%) Pepsin eingesetzt.

Bei der Simulation des Darms wird eine Anfangs-Gesamt-Enzymkonzentration bevorzugt (einschließlich Gallenextrakt/-salze), die 0,5 -12% beträgt, besonders bevorzugt 1 - 8%. Weiter bevorzugt ist dabei eine Anfangs-Konzentration von Pankreatin, Trypsin und Gallenextrakt/-salze von jeweils 0,3 - 3%, für die erste Verdünnung 0,1 - 0,8%, für die zweite Verdünnung 0,02 - 0,2%, für die dritte Verdünnung 0,005 - 0,1 %, und für die vierte Verdünnung 0,002 - 0,09%. Vorteilhaft sind dabei zwei Verdünnungen, bevorzugt sind drei oder vier Verdünnungen. Die Gesamt-Enzymkonzentration nimmt damit bevorzugt um mehr als 70% ab, besonders bevorzugt um mehr als 90%.

Konkrete Werte für eine vorteilhafte Darm-Simulation zeigt die folgende Tabelle.

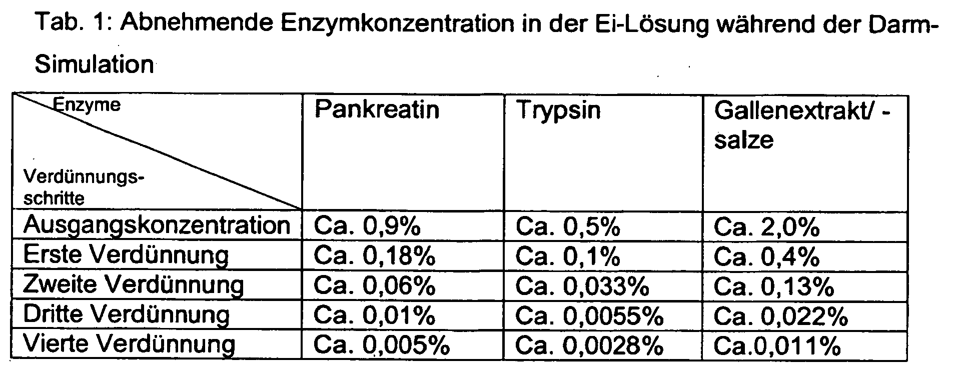

Auch nur Teile der Darmpassagen-Simulation reichen erfindungsgemäß aus, um einen Schlupf hervorzurufen. Ebenso reicht es erfindungsgemäß aus, nur Teile der Magen-Darm-Passage eines Schweins zu simulieren, insbesondere das Milieu des Mauls und der Speiseröhre nicht zu berücksichtigen; vorteilhaft kann jedoch auch dies noch vor der Magen-Simulation geschehen. Ebenso reicht es erfindungsgemäß aus, nur einzelne Enzyme (in der Magen-Simulation in absteigender Konzentration) einzusetzen.

Bei einer optionalen Simulation des Mauls wird bevorzugt 1-5% Amylase eingesetzt.

Als vorteilhafte Enzym-Beispiele können folgende Verdauungsstoffe, Salze, Enzymgemische oder Enzyme genannt werden, mit deren Hilfe erfindungsgemäß Larven aus den Eiern zum Schlüpfen gebracht werden:
1. Amylase des Schweins oder anderer Tiere, insbesondere in einer Konzentration zwischen 0,1 - 10%.
2. Pepsin des Schweins oder anderer Tiere, insbesondere in einer Konzentration zwischen 0,1 - 10%, vorteilhaft in einer stark angesäuerten PBS-Lösung (Salzpuffer) (pH - Wert insbesondere: 0,8 -3).
3. Gallenextrakt/ -salze des Schweins oder anderer Tiere, insbesondere in einer Konzentration zwischen 0,5 -12%.
4. Pankreatin des Schweins oder anderer Tiere, insbesondere in einer Konzentration zwischen 0,1 - 10%.
5. Trypsin des Schweins oder anderer Tiere, insbesondere in einer Konzentration zwischen 0,01 -1 %.

Dabei sind jeweils die Enzyme und Gallenextrakt/ -salze des Schweins bevorzugt. (Hier und im Folgenden wird unter einem Schwein bevorzugt ein Hausschwein verstanden.) - Gelöst werden die Enzyme und Verdauungsstoffe vorteilhaft in einer PBS-Lsg. (andere isotonische oder nichtisotonische Salzlösungen sind möglich). Vorzugsweise variiert der pH-Wert während der Simulation der Magen-Darm-Passage. Der pH-Wert liegt insbesondere zwischen 0,8 und 3,0 (bei Simulation der Magenpassage) und insbesondere zwischen 5 und 8 (bei Simulation der Darmpassage).

Inkubiert werden die Eier vorteilhaft in einem Klima- oder Wärmeschrank bei Temperaturen insbesondere zwischen 35 und 43°C, besonders bevorzugt 39,4°C, wobei vorteilhaft die Gaskonzentration, insbesondere der CO₂-Gehalt, eingestellt wird, wobei ein CO₂-Gehalt von 4-7%, besonders bevorzugt 5,6%, (Rest Luft) besonders vorteilhaft ist.

Vorteilhaft wird die Ei-Enzym-Mischung im Klima- oder Wärmeschrank auf einem Rüttler geschwenkt, wobei die Larven nach ca. 24 bis 168 Std. schlüpfen.

Im Folgenden wird die Erfindung anhand eines Ausführungsbeispiels erläutert, ohne aber diese dadurch unnötig einschränken zu wollen.

Beispiel einer erfindungsgemäß simulierten *in vitro* Magen-Darm-Passage, die zum Schlupf des ersten Larvalstadiums des Peitschenwurm *Trichuris suis* aus dem Ei führt.

Chemikalien- und Enzym-Lösungen, die bei dem Schlupf von *Trichris suis-*Larven aus den Eiern verwendet wurden (alle Lösungen wurden kurz vor dem Einsatz frisch hergestellt):
1. PBS-Puffer (Dulbecco's Phosphate-Buffered Saline W/O Ca / Mg): fertig gekaufter Puffer, der nicht verändert wird.
2. HCl-Lösung: 1 ml einer 25%igen HCl-Lösung wird in 49 ml PBS-Puffer (1.) gelöst.
3. Kaliummonohydrogenphosphat-Puffer: 1,9 g Kaliummonohydrogenphosphat werden in 48 ml PBS-Puffer (1.) gelöst.
4. Amylase: 0,5g Amylase wird in 12,5 ml PBS-Puffer (1.) gelöst.
5. Pepsin-Lösung: 1,25g Pepsin wird in 25 ml, der unter 2. hergestellten HCl-Lösung, gelöst.
6. Pankreatin-Trypsin-LÖsung: 0,225 g Pankreatin und 0,083g Trypsin werden in 12,5 ml PBS-Puffer (1.) gelöst. Pankreatin und Trypsin werden 1,5 h vor Verwendung in Lösung gebracht und 1 h auf einem Rotator, bei 39,4°C, gemischt. Dann wird die Lösung 0,5 h im Kühlschrank gelagert, damit größere Schwebstoffe sich absetzen können. Aus dem oberen Drittel wird Lösung für die Versuche entnommen.
7. Gallenextrakt/ salz -Lösung: 5 g Galenextrakt wird in 25 ml PBS-Puffer (1.) gelöst. Gallenextrakt wird 1,5 h vor Verwendung in Lösung gebracht und 1 h auf einem Rotator, bei 39,4°C, gemischt. Dann wird die Lösung 0,5 h im Kühlschrank gelagert, damit größere Schwebstoffe sich absetzen können. Aus dem oberen Drittel wird Lösung für die Versuche entnommen.
8. Atemluftgemisch: 94,4% Atemluft plus 5,6% CO₂.

**Tab. 2: Hersteller und Artikel-Nummer der verwendeten Lösungen, Chemikalien und Enzyme**

| | Lösung / Chemikalie | Hersteller | Artikel - Nummer |
|---|---|---|---|
| 1 | PBS-Puffer | Invitrogen | 14190169 |
| 2 | 25 %ige HCI-Lösung | Merck | 1.00316.1000 |
| 3 | Kaliummonohydrogenphosphat | Synopharm | 163690-0001 |
| 4 | Amylase | Sigma-Aldrich | A3176-500KU |
| 5 | Pepsin | Sigma-Aldrich | P-7000 25g |
| 6 | Pankreatin | Fluka | 76190 |
| 7 | Trypsin | Sigma-Aldrich | T4799-5G |
| 8 | Gallenextraktl -salze | Sigma-Aldrich | B8631-100G |
| 9 | Atemluftgemisch mit 5,6% CO2 | Linde | Sonderanfertigung |
| 10 | Ei - Urlösung | Parasite Technology | Batch PT-M32 |
| 11 | Aufgereinigte Ei - Lösung | Ovamed | 1602 |

**Tab. 3: Verwendete Laborgeräte**

| | Gerät | Hersteller | Typ |
|---|---|---|---|
| 1 | Zentrifuge | Hettich | Rotixa 50 S |
| 2 | Laminar Air flow | Holton | HB 2460 |
| 3 | Brutschrank | Binder | BD 53 |
| 4 | Rüttler | Kinematica | VXR S13 |
| 5 | Rotator | Stuart | SB2 |

### Durchführung:

### Tag 1

Aus der Ei-Urlösung wird die Ei-Lösung, mit einem pH-Wert um 7 und einer Ei-Zahl von ca. 500 embryonierten Eiern/ml hergestellt. Aus der Ei-Lösung werden 20 ml (ca. 10.000 embryonierte Eier) entnommen und in ein Falcon Röhrchen überführt.

In der Zentrifuge wird die Ei-Lösung 5 Minuten bei 500 U/min zentrifugiert (alle weiteren Zentrifugationsschritte werden bei dieser Einstellung durchgeführt), so dass die Eier durch die Zentrifugalkraft auf den Grund des Falcon Röhrchens absinken.

Das Falcon Röhrchen wird einer Außendesinfektion unterzogen und unter die Laminar-Air-Flow (LF) gebracht. Unter der LF werden 19,5 ml des Überstandes abpipettiert und verworfen (Zugabe und Entfernen von Lösungen findet nur unter der LF statt, ist aber nicht zwingend notwendig).

In das Falcon Röhrchen wird 0,5 ml PBS-Puffer und 1 ml der Amylase-Lösung zugegeben.

Das Falcon Röhrchen wird verschlossen und auf dem Rüttler, unter leichten Rüttelbewegungen (zwischen 50 und 100 U/min, wird während des Versuchs nicht verändert), bei 39,4°C im Brutschrank 0,5 h bebrütet.

Die Lösung wird zentrifugiert. 1,5 ml Überstand werden abpipettiert und verworfen. Es werden 1 ml der hergestellten Pepsin -Lösung und 1 ml der hergestellten HCl-Lösung zugegeben.

Das Falcon Röhrchen wird verschlossen und auf dem Rüttler, unter leichten Rüttelbewegungen, bei 39,4°C im Brutschrank 3 h bebrütet.

6 ml der Kaliummonohydrogenphosphat-Lösung wird hinzu pipettiert. Dadurch findet eine pH-Wert Erhöhung statt.

Die Lösung wird zentrifugiert. 7 ml Überstand wird abpipettiert und verworfen.

In die 1,5 ml neutrale Lösung werden 2,5 ml PBS-Puffer, 5 ml Pankreatin-Trypsin-Lösung und 1 ml Gallenextrakt/ -salz -Lösung zugegeben.

Ab jetzt wird **nach jedem Öffnen**, in den Luftraum des Falcon Röhrchens, über der Lösung das Atemluftgemisch mit 5,6% CO2 geblasen. Das Falcon Röhrchen wird verschlossen und auf dem Rüttler, bei 39,4°C im Brutschrank 3 h bebrütet.

Die Lösung wird auf 2 Falcon Röhrchen aufgeteilt und identisch weiterbehandelt. Das weitere Vorgehen wird jetzt an einem Falcon Röhrchen dargestellt.

Es werden 20 ml PBS-Puffer in das Falcon Röhrchen pipettiert. Das Falcon Röhrchen wird verschlossen und auf dem Rüttler, bei 39,4°C im Brutschrank 16 h bebrütet.

### Tag 2

Die Lösung wird zentrifugiert. 20 ml Überstand werden abpipettiert und verworfen. Es werden 10 ml PBS-Puffer zugegeben.

Das Falcon Röhrchen wird verschlossen und auf dem Rüttler, bei 39,4°C im Brutschrank 3 h bebrütet.

Die Lösung wird zentrifugiert. 13 ml Überstand werden abpipettiert und verworfen. Es werden 10 ml PBS-Puffer zugegeben.

Die Lösung wird zentrifugiert. 6 ml Überstand werden abpipettiert und verworfen.

Das Falcon Röhrchen wird verschlossen und auf dem Rüttler, bei 39,4°C im Brutschrank 3 h bebrütet.

Es werden 6 ml PBS-Puffer zugegeben.

Das Falcon Röhrchen wird verschlossen und auf dem Rüttler, bei 39,4°C im Brutschrank 16 h bebrütet.

### Tag 3

Die Lösung wird zentrifugiert. 6 ml Überstand werden abpipettiert und verworfen.

In dem Falcon Röhrchen befinden sich ca. 6 ml verdünnte Enzym-Lösung und ca. 5000 embryonierte Eier. Das Röhrchen wird auf dem Rüttler, bei 39,4°C im Brutschrank 24 h bebrütet.

### Tag 4

Kontrolle der Lösung auf geschlüpfte Larven. Das Röhrchen wird unter leichten Rüttelbewegungen, bei 39,4°C im Brutschrank 24 h bebrütet.

### Tag 5

Kontrolle der Lösung auf geschlüpfte Larven. Das Röhrchen wird auf dem Rüttler, bei 39,4°C im Brutschrank 24 h bebrütet.

### Tag 6

Kontrolle der Lösung auf geschlüpfte Larven. Das Röhrchen wird auf dem Rüttler, bei 39,4°C im Brutschrank 24 h bebrütet.

### Tag 7

Kontrolle der Lösung auf geschlüpfte Larven. Das Röhrchen wird auf dem Rüttler, bei 39,4°C im Brutschrank 24 h bebrütet.

### Tag 8

Kontrolle der Lösung auf geschlüpfte Larven. Das Röhrchen wird auf dem Rüttler, bei 39,4°C im Brutschrank 24 h bebrütet.

Vom vierten zum fünften Tag steigt die Anzahl der lebenden Larven von ca. 2% auf ca. 10%. Ab dem sechsten Tag verringert sich die Anzahl der lebenden Tiere. Die Rate der toten Tiere steigt vom dritten (2-4%) bis zum achten Tag (über 50%) kontinuierlich an. Es ist davon auszugehen, dass alle toten Tiere aktiv geschlüpft sind und somit beim Schlupf am Leben waren.

## Patentansprüche

1. Verfahren zum Nachweis der Viabilität von *Trichuris suis -* Eiern, **dadurch gekennzeichnet, dass** man den Durchgang der Eier durch die Magen-Darm-Passage eines Schweins *in vitro* simuliert hinsichtlich zeitlicher Abfolge und Milieu, nämlich zunächst das Magen-Milieu und anschließend in absteigender Enzym- und Salz-Konzentration das Darm-Milieu, und damit die Larven aus den Eiern zum Schlüpfen bringt.

2. Verfahren nach Anspruch 1, **dadurch gekennzeichnet, dass** die Simulation der Magen-Darm-Passage eines Schweins erfolgt durch Zugabe von entsprechenden Enzymen und Inkubation bei erhöhter Temperatur, wobei im einzelnen insbesondere folgende Parameter eingestellt werden:
- Enzyme, in der Darmsimulation mit abnehmender Konzentration,
- pH-Wert,
- Wärme,
- evtl. unterschiedliche Gaskonzentration im Klimaschrank,
- Rüttelbewegungen,
- Salzlösungen, in der Darmsimulation mit abnehmender Konzentration,

3. Verfahren nach Anspruch 1 oder 2, **dadurch gekennzeichnet, dass** die Simulation in einem flüssigen oder halbfesten Milieu, insbesondere in einer Lösung geschieht.

4. Verfahren nach einem der Ansprüche 1-3, **dadurch gekennzeichnet, dass** das Milieu zur Simulation der Darm-Passage eine über die Zeit abnehmende Enzymkonzentration aufweist.

5. Verfahren nach einem der Ansprüche 1-4, **dadurch gekennzeichnet, dass** nur Teile der Magen-Darm-Passage eines Schweins simuliert werden.

6. Verfahren nach einem der Ansprüche 1-5, **dadurch gekennzeichnet, dass** man im Milieu Enzyme einsetzt, insbesondere Verdauungsstoffe, Salze, Enzymgemische oder Enzyme, bevorzugt Amylase des Schweins oder anderer Tiere, insbesondere in einer Konzentration zwischen 0,1 - 10%, Pepsin des Schweins oder anderer Tiere, insbesondere in einer Konzentration zwischen 0,1
- 10%, vorteilhaft in einer stark angesäuerten PBS-Lösung (Salzpuffer) (pH - Wert insbesondere: 0,8 -3), Gallenextrakt/ -salze des Schweins oder anderer Tiere, insbesondere in einer Konzentration zwischen 0,5 - 12%, Pankreatin des Schweins oder anderer Tiere, insbesondere in einer Konzentration zwischen 0,1
- 10%, Trypsin des Schweins oder anderer Tiere, insbesondere in einer Konzentration zwischen 0,01 -1 %, wobei Enzyme und Salze bevorzugt brei folgenden pH-Werten eingesetzt werden:
| Enzym | pH-Wert |
|---|---|
| Amylase | 5 - 8 |
| Pepsin | 0,3 - 3 |
| Pankreatin | 5 - 8 |
| Trypsin | 5 - 8 |
| Gallenextrakt/-salze | 5 - 8 |

7. Verfahren nach einem der Ansprüche 1-6, **dadurch gekennzeichnet, dass** man im Milieu Enzyme des Schweins einsetzt.

8. Verfahren nach einem der Ansprüche 1-7, **dadurch gekennzeichnet, dass** der pH-Wert des Milieus zwischen 0,8 und 3,0 (bei Simulation der Magenpassage) und zwischen 5 und 8 (bei Simulation der Darmpassage) eingestellt wird.

9. Verfahren nach einem der Ansprüche 1-8, **dadurch gekennzeichnet, dass** die Simulation bei Temperaturen zwischen 35 und 43°C, besonders bevorzugt bei 39,4°C durchgeführt wird.

10. Verfahren nach einem der Ansprüche 1-9, **dadurch gekennzeichnet, dass** der CO₂-Gehalt über dem Milieu auf 4-7%, besonders bevorzugt auf 5,6% eingestellt wird (Rest Luft).

11. Verfahren nach einem der Ansprüche 1-10, **dadurch gekennzeichnet, dass** die Eier zunächst einem simulierten Schweinemagen-Milieu ausgesetzt werden, dass dann die Eier von diesem Milieu getrennt werden, insbesondere durch Zentrifugieren, dass darauf die so behandelten Eier einem simulierten Schweinedarm-Milieu ausgesetzt werden, wobei die Enzymkonzentration dieses Milieus mit der Zeit abnehmend eingestellt wird, insbesondere durch Verdünnen, wobei diese Behandlungen vorteilhaft unter einer CO₂-haltigen Atmosphäre durchgeführt werden, mit einem CO₂-Gehalt von 4-7%, bevorzugt auf 5,6%, und bei einer Temperatur von 35 - 43°C, bevorzugt bei 39,4°C.

## Claims

1. A method of detecting the viability of *Trichuris suis* eggs, **characterized in that** the passage of the eggs through the gastrointestinal tract of a pig is simulated *in vitro* with regard to chronological sequence and medium, viz. initially the gastric medium and subsequently, in decreasing enzyme and salt concentration, the intestinal medium, and thereby causes the larvae to hatch from the eggs.

2. A method according to claim 1, **characterized in that** the gastrointestinal tract of a pig is simulated by addition of appropriate enzymes and incubation at elevated temperature and specifically the following parameters in particular are set:
- enzymes, with decreasing concentration in the intestinal simulation,
- pH,
- heat,
- possibly different gas concentration in a climate chamber,
- agitating movements,
- salt solutions, with decreasing concentration in the intestinal simulation.

3. A method according to claim 1 or 2, **characterized in that** the simulation takes place in a liquid or semisolid medium, more particularly in a solution.

4. A method according to any one of claims 1-3, **characterized in that** the medium for simulating the intestinal passage has an enzyme concentration that decreases over time.

5. A method according to any one of claims 1-4, **characterized in that** only parts of the gastro-intestinal tract of a pig are simulated.

6. A method according to any one of claims 1-5, **characterized in that** enzymes are used in the medium, more particularly digestive substances, salts, enzyme mixtures or enzymes, preferably amylase, from the pig or other animals, more particularly in a concentration between 0.1 - 10%, pepsin from the pig or other animals, more particularly in a concentration between 0.1 - 10%, advantageously in a highly acidified PBS solution (salt buffer) (pH more particularly: 0.8 - 3), bile extract/salts from the pig or other animals, more particularly in a concentration between 0.5 - 12%, pancreatin from the pig or other animals, more particularly in a concentration between 0.1 - 10%, trypsin from the pig or other animals, more particularly in a concentration between 0.01 - 1%, wherein enzymes and salts are preferably used at the following pH values:
| Enzyme | pH value |
|---|---|
| Amylase | 5 - 8 |
| Pepsin | 0.3 - 3 |
| Pancreatin | 5 - 8 |
| Trypsin | 5 - 8 |
| Bile extract/salts | 5 - 8 |

7. A method according to any one of claims 1-6, **characterized in that** enzymes from the pig are used in the medium.

8. A method according to any one of claims 1-7, **characterized in that** the pH of the medium is set between 0.8 and 3.0 (for simulation of the gastric passage) and between 5 and 8 (for simulation of the intestinal passage).

9. A method according to any one of claims 1-8, **characterized in that** the simulation is carried out at temperatures between 35 and 43°C, with particular preference at 39.4°C.

10. A method according to any one of claims 1-9, **characterized in that** the CO₂ content above the medium is set to 4-7% and more preferably to 5.6% (the remainder being air).

11. A method according to any one of claims 1-10, **characterized in that** the eggs are initially exposed in a simulated porcine gastric medium, **in that** the eggs are then separated from this medium, more particularly by centrifugation, **in that** the eggs thus treated are exposed in a simulated porcine intestinal medium, the enzyme concentration of this medium being set, more particularly by dilution, to decrease over time, these treatments advantageously being carried out under a CO₂-containing atmosphere, using a CO₂ content of 4-7%, preferably of 5.6%, and at a temperature of 35-43°C, preferably at 39.4°C.

## Revendications

1. Procédé pour détecter la viabilité d'oeufs de *Trichuris suis,* **caractérisé en ce qu'**on simule *in vitro* le passage des oeufs à travers le passage gastro-intestinal d'un porc, pour ce qui concerne son déroulement dans le temps et son milieu, plus précisément d'abord le milieu gastrique, puis, pour une concentration décroissante des enzymes et des sels, le milieu intestinal, et ainsi on fait éclore les larves à partir des oeufs.

2. Procédé selon la revendication 1, **caractérisé en ce que** la simulation du passage gastro-intestinal d'un porc s'effectue par addition d'enzymes appropriées et incubation à une haute température, ce à l'occasion de quoi on ajuste en détail en particulier les paramètres suivantes :
- les enzymes, dans la simulation intestinale à concentration décroissante,
- le pH,
- la chaleur,
- éventuellement la concentration variable du gaz en armoire climatique,
- les mouvements de secousses,
- les solutions salées, dans la simulation intestinale à concentration décroissante.

3. Procédé selon la revendication 1 ou 2, **caractérisé en ce que** la simulation a lieu dans un milieu liquide ou semi-solide, en particulier en solution.

4. Procédé selon l'une des revendications 1 à 3, **caractérisé en ce que** le milieu pour la simulation du passage intestinal présente une concentration d'enzymes qui décroît dans le temps.

5. Procédé selon l'une des revendications 1 à 4, **caractérisé en ce que** seules sont simulées des parties du passage gastro-intestinal d'un porc.

6. Procédé selon l'une des revendications 1 à 5, **caractérisé en ce qu'**on utilise dans le milieu des enzymes, en particulier des substances digestives, des selles, des mélanges d'enzymes ou des enzymes, de préférence l'amylase du porc ou d'autres animaux, en particulier à une concentration comprise entre 0,1 et 10%, la pepsine du porc ou d'autres animaux, en particulier à une concentration comprise entre 0,1 et 10%, avantageusement dans une solution de PBS (tampon sel) fortement acidifiée (pH en particulier de 0,8 à 3), les extraits/sels biliaires du porc ou d'autres animaux, en particulier à une concentration comprise entre 0,5 et 12%, la pancréatine du porc ou d'autres animaux, en particulier à une concentration de 0,1 à 10%, la trypsine du porc ou d'autres animaux, en particulier à une concentration comprise entre 0,01 et 1%, les enzymes et sels étant de préférence utilisés aux pH suivantes :
| Enzyme | pH |
|---|---|
| Amylase | 5 - 8 |
| Pepsine | 0,3 - 3 |
| Pancréatine | 5 - 8 |
| Trypsine | 5 - 8 |
| Extrait/sels biliaires | 5 - 8 |

7. Procédé selon l'une des revendications 1 à 6, **caractérisé en ce qu'**on utilise dans le milieu des enzymes du porc.

8. Procédé selon l'une des revendications 1 à 7, **caractérisé en ce que** le pH du milieu est ajusté à une valeur comprise entre 0,8 et 3,0 (lors de la simulation du passage gastrique) et entre 5 et 8 (lors de la simulation du passage intestinal).

9. Procédé selon l'une des revendications 1 à 8, **caractérisé en ce que** la simulation est effectuée à des températures comprises entre 35 et 43°C, d'une manière particulièrement préférée à 39,4°C.

10. Procédé selon l'une des revendications 1 à 9, **caractérisé en ce que** la teneur en CO₂ au-dessus du milieu est ajustée à 4-7% et d'une manière particulièrement préférée à 5,6% (le reste étant constitué d'air).

11. Procédé selon l'une des revendications 1 à 10, **caractérisé en ce que** les oeufs sont d'abord exposés à un milieu gastrique de porc simulé, que l'on sépare ensuite les oeufs de ce milieu, notamment par centrifugation, que l'on expose ensuite les oeufs ainsi traités à un milieu intestinal de porc simulé, la concentration des enzymes dans ce milieu étant ajustée de façon à décroître dans le temps, en particulier par dilution, ces traitements étant avantageusement mis en oeuvre sous une atmosphère contenant du CO₂, avec une teneur en CO₂ de 4-7%, de préférence de 5,6%, et à une température de 35 à 43°C, de préférence à 39,4°C.
